# EUROPEAN PATENT SPECIFICATION

(11) **EP 3 093 350 B1**
(45) Date of publication and mention of the grant of the patent: **19.06.2019**
(21) Application number: 16158591.4
(22) Date of filing: 22.10.2009
(51) Int. Cl.: C12Q 1/6883, C12Q 1/6886

(54) **COMPOSITIONS AND METHODS FOR DETECTING MUTATIONS IN JAK2 NUCLEIC ACID**
ZUSAMMENSETZUNGEN UND VERFAHREN ZUR DETEKTION VON MUTATIONEN IN JAK2-NUKLEINSÄURE
COMPOSITIONS ET PROCÉDÉS POUR DÉTECTER DES MUTATIONS DANS UN ACIDE NUCLÉIQUE JAK2

(30) Priority: 31.10.2008 US 110501 P; 15.07.2009 US 503318
(43) Date of publication of application: 16.11.2016
(62) Divisional of application: 14161006.3
(73) Proprietor: Quest Diagnostics Investments Incorporated, Wilmington, DE 19899 (US)
(72) Inventor: ALBITAR, Maher, San Juan Capistrano, CA 92675 (US); MA, Wanlong, Aliso Viejo, CA 92656 (US)
(74) Representative: Hoffmann Eitle

(56) References cited:
- US-A1- 2007 248 961
- TEFFERI A: "JAK and MPL mutations in myeloid malignancies", LEUKEMIA AND LYMPHOMA, HARWOOD ACADEMIC PUBLISHERS, CHUR, CH, vol. 49, no. 3, 1 March 2008 (2008-03-01), pages 388-397, XP009157130, ISSN: 1042-8194, DOI: 10.1080/10428190801895360
- BESSES CARLOS ET AL: "JAK2 mutations at exon 12 and 14 in polycythemia vera and idiopathic erythrocytosis: Incidence and correlation with clinical characteristics", BLOOD, AMERICAN SOCIETY OF HEMATOLOGY, US, vol. 110, no. 11, part 1, 1 November 2007 (2007-11-01), page 746A, XP009157146, ISSN: 0006-4971
- LI SAI ET AL: "Clonal heterogeneity in polycythemia vera patients with JAK2 exon12 and JAK2-V617F mutations", BLOOD, vol. 111, no. 7, April 2008 (2008-04), pages 3863-3866, XP002670896, ISSN: 0006-4971
- SCOTT L M ET AL: "JAK2 exon 12 mutations in polycythemia vera and idiopathic erythrocytosis", NEW ENGLAND JOURNAL OF MEDICINE 20070201 US LNKD- DOI:10.1056/NEJMOA065202, vol. 356, no. 5, 1 February 2007 (2007-02-01), pages 459-468, XP002670897, ISSN: 0028-4793
- SMITH C A ET AL: "The saga of JAK2 mutations and translocations in hematologic disorders: pathogenesis, diagnostic and therapeutic prospects, and revised World Health Organization diagnostic criteria for myeloproliferative neoplasms", HUMAN PATHOLOGY, SAUNDERS, PHILADELPHIA, PA, US, vol. 39, no. 6, 1 June 2008 (2008-06-01), pages 795-810, XP022693901, ISSN: 0046-8177, DOI: 10.1016/J.HUMPATH.2008.02.004 [retrieved on 2008-06-03]
- LEE TAI-SUNG ET AL: "Structural effects of clinically observed mutations in JAK2 exons 13-15: comparison with V617F and exon 12 mutations", BMC STRUCTURAL BIOLOGY, BIOMED CENTRAL LTD., LONDON, GB, vol. 9, no. 1, 10 September 2009 (2009-09-10), page 58, XP021058180, ISSN: 1472-6807, DOI: 10.1186/1472-6807-9-58
- MA WANLONG ET AL: "Mutation Profile of JAK2 Transcripts in Patients with Chronic Myeloproliferative Neoplasias", JOURNAL OF MOLECULAR DIAGNOSTICS, AMERICAN SOCIETY FOR INVESTIGATIVE PATHOLOGY, BETHESDA, MD, US, vol. 11, no. 1, 1 January 2009 (2009-01-01), pages 49-53, XP009115055, ISSN: 1525-1578, DOI: 10.2353/JMOLDX.2009.080114
- CONSTANTINESCU ET AL: "Mining for JAK-STAT mutations in cancer", TRENDS IN BIOCHEMICAL SCIENCES, ELSEVIER, AMSTERDAM, NL, vol. 33, no. 3, 4 March 2008 (2008-03-04), pages 122-131, XP022510481, ISSN: 0968-0004
- KOPPIKAR PRIYA ET AL: "JAK2 and MPL mutations in myeloproliferative neoplasms", ACTA HAEMATOLOGICA, S. KARGER, BASEL, CH, vol. 119, no. 4, 1 January 2008 (2008-01-01), pages 218-225, XP009157128, ISSN: 0001-5792, DOI: 10.1159/000140634
- R KRALOVICS: "Genetic complexity of myeloproliferative neoplasms", LEUKEMIA, vol. 22, no. 10, 1 October 2008 (2008-10-01), pages 1841-1848, XP055020868, ISSN: 0887-6924, DOI: 10.1038/leu.2008.233
- FEENSTRA JELENA D MILOSEVIC ET AL: "Whole-exome sequencing identifies novel MPL and JAK2 mutations in triple-negative myeloproliferative neoplasms", BLOOD, vol. 127, no. 3, 21 January 2016 (2016-01-21), pages 325-332, ISSN: 0006-4971(print)

## Description

### FIELD OF THE INVENTION

This invention relates to the field of disease detection and more specifically to compositions and diagnostic methods useful for patients having hematopoietic disorders such as a myeloproliferative disease.

### BACKGROUND OF THE INVENTION

The following discussion of the background of the invention is merely provided to aid the reader in understanding the invention and is not admitted to describe or constitute prior art to the invention.

The Janus kinases are a family of tyrosine kinases that play a role in cytokine signaling. For example, JAK2 kinase acts as an intermediary between membrane-bound cytokine receptors such as the erythropoietin receptor (EpoR), and down-stream members of the signal transduction pathway such as STAT5 (Signal Transducers and Activators of Transcription protein 5). *See, e.g.,* Schindler, C.W., J. Clin Invest. 109:1133-1137 (2002); Tefferi and Gilliland, Mayo Clin. Proc. 80:947-958 (2005); Giordanetto and Kroemer, Protein Engineering, 15(9):727-737 (2002). JAK2 is activated when cytokine receptor/ligand complexes phosphorylate the associated JAK2 kinase. *Id.* JAK2 can then phosphorylate and activate its substrate molecule, for example STAT5, which enters the nucleus and interacts with other regulatory proteins to affect transcription. *Id*.; Nelson, M.E., and Steensma, D.P., Leuk. Lymphoma 47:177-194 (2006).

Certain hematopoietic diseases including non-CML myeloproliferative diseases (MPDs) such as polycythemia vera (PV), essential thrombocythemia (ET), and chronic idiopathic myelofibrosis (IMF) and as of yet unclassified myeloproliferative diseases (MPD-NC) are characterized by an aberrant increase in blood cells. *See e*.*g*., Vainchenker and Constantinescu, Hematology (American Society of Hematology), 195-200 (2005). This increase is generally initiated by a spontaneous mutation in a multipotent hematopoietic stem cell located in the bone marrow. *Id.* Due to the mutation, the stem cell produces far more blood cells of a particular lineage than normal, resulting in the overproduction of cells such as erythroid cells, megakaryocytes, granulocytes and monocytes. Some symptoms common to patients with MPD include enlarged spleen, enlarged liver, elevated white, red and/or platelet cell count, blood clots (thrombosis), weakness, dizziness and headache. Diseases such as PV, ET and IMF may presage leukemia, however the rate of transformation (*e*.*g*., to blast crisis) differs with each disease. *Id.* It has long been postulated that perturbation of protein tyrosine kinase (PTK) signaling by mutations and other genetic alterations is associated with MPDs. Mutant PTKs such as, for example, Janus kinase 2 (JAK2) gene mutations, can lead to constitutive activity in patients with MPDs.

The specific gene and concomitant mutation or mutations responsible for many MPDs is not known. However, a mutation in the Janus kinase 2 (JAK2) gene, a cytoplasmic, nonreceptor tyrosine kinase, has been identified in a number of MPDs. The discovery of the JAK2 V617F mutation was a milestone in unveiling the molecular pathogenesis of MPDs. For example, this mutation has been reported in up to 97% of patients with PV, and in greater than 40% of patients with either ET or IMF. *See e*.*g*., Baxter et al., Lancet 365:1054-1060 (2005); James et al., Nature 438:1144-1148 (2005); Zhao, et al., J. Biol. Chem. 280(24):22788-22792 (2005); Levine et al., Cancer Cell, 7:387-397 (2005); Kralovics, et al., New Eng. J. Med. 352(17):1779-1790 (2005); Jones, et al., Blood 106:2162-2168 (2005); Steensma, et al., Blood 106:1207-2109 (2005).

A variety of different approaches and a large body of evidence suggest that, when present, the JAK2 V617F mutation contributes to the pathogenesis of MPD. *See e*.*g*., Kaushansky, Hematology (Am Soc Hematol Educ Program), 533-7 (2005). The mutation has been detected from blood samples, bone marrow and buccal samples (*see*, *e*.*g*., Baxter et al., Lancet 365:1054-1060 (2005); James et al., Nature 438:1144-1148 (2005); Zhao, et al., J. Biol. Chem. 280(24):22788-22792 (2005); Levine et al., Cancer Cell, 7:387-397 (2005); Kralovics, et al., New Eng. J. Med. 352(17):1779-1790 (2005)), and homozygous and heterozygous cell populations have been reported in MPD patients. Baxter et al., Lancet 365:1054-1060 (2005).

The JAK2 V617F substitution, which is located in the pseudokinase domain of JAK2, relieves the auto-inhibition of its kinase activity, leading to a constitutively active kinase and augments downstream JAK2-STAT signaling pathways (*see e*.*g*., Saharinen et al., Mol Cell Biol 20:3387-3395 (2000); Saharinen et al., Mol. Biol Cell 14:1448-1459 (2003). Other *JAK2* mutations in humans including translocations, point mutations, deletions, and insertions have been reported. *See e*.*g*., Scott et al., N Engl J Med 356:459-468 (2007); Li et al., Blood 111:3863-3866 (2008).

### SUMMARY OF THE INVENTION

The invention is based on the identification of previously unknown mutations in the JAK2 gene and JAK2 protein. Specifically, the JAK2 gene and protein mutation is t2160g and F556V, respectively. The invention further provides compositions and methods useful in the diagnosis and prognosis of hematopoietic diseases including, for example, myeloproliferative diseases. The invention is as set out in the claims.

In one aspect, the invention provides an isolated nucleic acid of at least 17 nucleotides of SEQ ID NO: 1, in which the fragment has the mutation t2160g, and the isolated nucleic acid is less than 5000 nucleotides. In one embodiment, the isolated nucleic acid includes at least one additional mutation shown in Table 2. In another embodiment, the isolated nucleic acid is labeled with a detectable label.

In a second aspect, the invention provides a polypeptide of at least 10 contiguous amino acids of SEQ ID NO: 2 in which the polypeptide has the mutation F556V, and the polypeptide is less than 1100 amino acids. In one embodiment, the polypeptide includes at least one additional mutation shown in Table 2. In another embodiment, the fragment is labeled with a detectable label.

In another aspect, the invention provides a method for detecting a hematopoietic disease comprising detecting the presence or absence of a JAK2 mutation using a biological sample obtained from an individual, comprising: a) evaluating a sample from the individual for the presence or absence of a mutation in JAK2 nucleic acid or JAK2 polypeptide, wherein said mutation in the JAK2 nucleic acid is t2160g, and the mutation in the JAK2 polypeptide is the amino acid substitution F556V; and b) identifying said individual as having a hematopoietic disease when said JAK2 nucleic acid or JAK2 polypeptide, respectively, comprises said mutation.

In some embodiments of any of the above aspects of the invention, the JAK2 nucleic acid is RNA. In other embodiments of any of the above aspects of the invention, the presence or absence of one or more mutations may be determined relative to SEQ ID NO: 1. In certain embodiments of any of the above aspects of the invention, the JAK2 nucleic acid includes two or more of the mutations shown in Table 2.

In still other embodiments of any of the above aspects of the invention, evaluating or determining the presence or absence of said mutation in a JAK2 nucleic acid sample includes amplifying JAK2 nucleic acid and hybridizing the amplified JAK2 nucleic acid with a detection oligonucleotide that is capable of specifically detecting a JAK2 nucleic acid mutant sequence under hybridization conditions. In other embodiments of any of the above aspects of the invention, evaluating or determining the presence or absence of said mutation in a JAK2 nucleic acid sample includes amplifying JAK2 nucleic acid and performing direct sequencing analysis of the amplified nucleic acid.

In some embodiments of the above aspects of the invention, the presence or absence of one or more mutations may be determined relative to SEQ ID NO: 2. In certain embodiments of the above aspects, the JAK2 polypeptide includes 2 or more of the mutations shown in Table 2. In certain embodiments of the above aspects, evaluating a sample or determining the presence or absence of said mutation in a JAK2 polypeptide sample includes using an antibody that specifically binds to the mutated JAK2 polypeptide.

In some embodiments of any of the above aspects of the invention, the JAK2 nucleic acid and/or polypeptide sample is from a biological fluid from the patient, preferably the sample is blood, serum, or plasma.

In certain embodiments of any of the above aspects of the invention, the disease is a myeloproliferative disease; more preferably, the myeloproliferative disease is polycythemia vera, essential thrombocythemia, idiopathic myelofibrosis, or an unclassified myeloproliferative disease.

The term "neoplastic disease" refers to a condition characterized by an abnormal growth of new cells such as a tumor. A neoplasm includes solid and non-solid tumor types such as a carcinoma, sarcoma, leukemia and the like. A neoplastic disease may be malignant or benign.

The term "myeloproliferative disease (MPD)" or "myeloproliferative disorder" is meant to include non-lymphoid dysplastic or neoplastic conditions arising from a hematopoietic stem cell or its progeny. "MPD patient" includes a patient who has been diagnosed with an MPD. "Myeloproliferative disease" is meant to encompass the specific, classified types of myeloproliferative diseases including polycythemia vera (PV), essential thrombocythemia (ET) and idiopathic myelofibrosis (IMF). Also included in the definition are hypereosinophilic syndrome (HES), chronic neutrophilic leukemia (CNL), myelofibrosis with myeloid metaplasia (MMM), chronic myelomonocytic leukemia (CMML), juvenile myelomonocytic leukemia, chronic basophilic leukemia, chronic eosinophilic leukemia, and systemic mastocytosis (SM). "Myeloproliferative disease" is also meant to encompass any unclassified myeloproliferative diseases (UMPD or MPD-NC).

As used herein the terms "diagnose" or "diagnosis" or "diagnosing" refer to distinguishing or identifying a disease, syndrome or condition or distinguishing or identifying a person having a particular disease, syndrome or condition.

"Determining a prognosis" as used herein refers to the process in which the course or outcome of a condition in a patient is predicted. The term "prognosis" does not refer to the ability to predict the course or outcome of a condition with 100% accuracy. Instead, the term refers to identifying an increased or decreased probability that a certain course or outcome will occur in a patient exhibiting a given condition/marker, when compared to those individuals not exhibiting the condition. The nature of the prognosis is dependent upon the specific disease and the condition/marker being assessed. For example, a prognosis may be expressed as the amount of time a patient can be expected to survive, the likelihood that the disease goes into remission, or to the amount of time the disease can be expected to remain in remission.

As used herein, the term "treatment," "treating," or "treat" refers to care by procedures or application that are intended to relieve illness or injury. Although it is preferred that treating a condition or disease such as a myeloproliferative disease will result in an improvement of the condition, the term treating as used herein does not indicate, imply, or require that the procedures or applications are at all successful in ameliorating symptoms associated with any particular condition. Treating a patient may result in adverse side effects or even a worsening of the condition which the treatment was intended to improve.

By "subject" is meant a human or any other animal which contains a JAK2 gene that can be amplified using the primers and methods described herein. A subject can be a patient, which refers to a human presenting to a medical provider for diagnosis or treatment of a disease. A human includes pre and post natal forms.

As used herein, the term "patient" refers to one who receives medical care, attention or treatment. As used herein, the term is meant to encompass a person diagnosed with a disease such as myeloproliferative disease as well as a person who may be symptomatic for a disease but who has not yet been diagnosed.

The term "sample" or "patient sample" is meant to include biological samples such as tissues and bodily fluids. "Bodily fluids" may include, but are not limited to, blood, serum, plasma, saliva, cerebral spinal fluid, pleural fluid, tears, lactal duct fluid, lymph, sputum, urine, amniotic fluid, and semen. A sample may include a bodily fluid that is "acellular." An "acellular bodily fluid" includes less than about 1% (w/w) whole cellular material. Plasma or serum are examples of acellular bodily fluids. A sample may include a specimen of natural or synthetic origin.

The term "nucleic acid" or "nucleic acid sequence" refers to an oligonucleotide, nucleotide or polynucleotide, and fragments or portions thereof, which may be single or double stranded, and represent the sense or antisense strand. A nucleic acid may include DNA or RNA, and may be of natural or synthetic origin. For example, a nucleic acid may include mRNA, genomic DNA or cDNA. Nucleic acid may include nucleic acid that has been amplified (*e*.*g*., using polymerase chain reaction). The convention "NTwt###NTmut" is used to indicate a mutation that results in the wild-type nucleotide NTwt at position ### in the nucleic acid being replaced with mutant NTmut.

For the JAK2 nucleic acid sequence, a "mutation" means a JAK2 nucleic acid sequence that includes at least one nucleic acid variation as compared to reference sequence GenBank accession number NM_004972 (SEQ ID NO: 1). A mutation in JAK2 nucleic acid may result in a change in the encoded polypeptide sequence or the mutation may be silent with respect to the encoded polypeptide sequence. A change in an amino acid sequence may be determined as compared to SEQ ID NO: 2, as a reference amino acid sequence.

The term "zygosity status" as used herein refers to a sample, a cell population, or an organism as appearing heterozygous, homozygous, or hemizygous as determined by testing methods known in the art and described herein. The term "zygosity status of a nucleic acid" means determining whether the source of nucleic acid appears heterozygous, homozygous, or hemizygous. The "zygosity status" may refer to differences in a single nucleotide in a sequence. In some methods, the zygosity status of a sample with respect to a single mutation may be categorized as homozygous wild-type, heterozygous (*i*.*e*., one wild-type allele and one mutant allele), homozygous mutant, or hemizygous (*i*.*e*., a single copy of either the wild-type or mutant allele). Because direct sequencing of plasma or cell samples as routinely performed in clinical laboratories does not reliably distinguish between hemizygosity and homozygosity, in some aspects, these classes are grouped. For example, samples in which no or a minimal amount of wild-type nucleic acid is detected are termed "hemizygous/homozygous mutant." In some aspects, a "minimal amount" may be between about 1-2%. In other aspects, a minimal amount may be between about 1-3%. In still other aspects, a "minimal amount" may be less than 1%.

The term "substantially all" as used herein means at least about 60%, about 70%, about 80%, about 90%, about 95%, about 99%, or 100%.

"Substantially pure" as used herein in the context of nucleic acid represents at least 50%, at least 60%, at least 70%, at least 80%, at least 90%, at least 95% or at least 99% of the nucleic acid in a sample. The nucleic acid sample may exist in solution or as a dry preparation.

By "isolated", when referring to a nucleic acid (e.g., an oligonucleotide such as RNA, DNA, or a mixed polymer) is meant a nucleic acid that is apart from a substantial portion of the genome in which it naturally occurs and/or is substantially separated from other cellular components which naturally accompany such nucleic acid. For example, any nucleic acid that has been produced synthetically (e.g., by serial base condensation) is considered to be isolated. Likewise, nucleic acids that are recombinantly expressed, cloned, produced by a primer extension reaction (e.g., PCR), or otherwise excised from a genome are also considered to be isolated.

As used herein, a "fragment" means a polynucleotide that is at least about 15, 20, 25, 30, 35, 40, 45, 50, 75, 100, 200, 300, 400, 500, 1000 nucleotides or more in length.

"Specific hybridization" is an indication that two nucleic acid sequences share a high degree of complementarity. Specific hybridization complexes form under permissive annealing conditions and remain hybridized after any subsequent washing steps. Permissive conditions for annealing of nucleic acid sequences are routinely determinable by one of ordinary skill in the art and may occur, for example, at 65°C in the presence of about 6×SSC. Stringency of hybridization may be expressed, in part, with reference to the temperature under which the wash steps are carried out. Such temperatures are typically selected to be about 5°C to 20°C lower than the thermal melting point (Tm) for the specific sequence at a defined ionic strength and pH. The Tm is the temperature (under defined ionic strength and pH) at which 50% of the target sequence hybridizes to a perfectly matched probe. Equations for calculating Tm and conditions for nucleic acid hybridization are known in the art.

By "substantially complementary" is meant that two sequences that will specifically hybridize. The skilled artisan will understand that substantially complementary sequences need not hybridize along their entire length.

Oligonucleotides used as primers or probes for specifically amplifying (*i*.*e*., amplifying a particular target nucleic acid sequence) or specifically detecting (*i*.*e*., detecting a particular target nucleic acid sequence) a target nucleic acid generally are capable of specifically hybridizing to the target nucleic acid.

The term "oligonucleotide" is understood to be a molecule that has a sequence of bases on a backbone comprised mainly of identical monomer units at defined intervals. The bases are arranged on the backbone in such a way that they can enter into a bond with a nucleic acid having a sequence of bases that are complementary to the bases of the oligonucleotide. The most common oligonucleotides have a backbone of sugar phosphate units. A distinction may be made between oligodeoxyribonucleotides that do not have a hydroxyl group at the 2' position and oligoribonucleotides that have a hydroxyl group in this position. Oligonucleotides also may include derivatives, in which the hydrogen of the hydroxyl group is replaced with organic groups, *e*.*g*., an allyl group. Oligonucleotides of the method which function as primers or probes are generally at least about 10-15 nucleotides long and more preferably at least about 15 to 25 nucleotides long, although shorter or longer oligonucleotides may be used in the method. The exact size will depend on many factors, which in turn depend on the ultimate function or use of the oligonucleotide. The oligonucleotide may be generated in any manner, including, for example, chemical synthesis, DNA replication, reverse transcription, PCR, or a combination thereof. The oligonucleotide may be modified. For example, the oligonucleotide may be labeled with an agent that produces a detectable signal (*e*.*g*., a fluorophore).

The term "detectable label" as used herein refers to a molecule or a compound or a group of molecules or a group of compounds associated with a nucleic acid or a polypeptide and is used to identify the nucleic acid or the polypeptide. In some cases, the detectable label may be detected directly. In other cases, the detectable label may be a part of a binding pair, which can then be subsequently detected. Signals from the detectable label may be detected by various means and will depend on the nature of the detectable label. Detectable labels may be isotopes, fluorescent moieties, colored substances, and the like. Examples of means to detect detectable label include but are not limited to spectroscopic, photochemical, biochemical, immunochemical, electromagnetic, radiochemical, or chemical means, such as fluorescence, chemifluoresence, or chemiluminescence, or any other appropriate means.

By "antibody that specifically binds to the mutated JAK2 polypeptide" is meant that the antibody preferentially binds to mutated JAK2 polypeptide and not to the wild type JAK2 polypeptide. Preferential binding of the antibody is meant to include at least 90% of the times the antibody will bind to mutated JAK2 polypeptide and discriminate between mutated and wild type JAK2 polypeptides.

As used herein, the term "activation domain" in reference to JAK2 refers generally to a domain involved in cell activation such as, for example, cell proliferation. An example of an activation domain is a kinase or pseudokinase domain.

As used herein, the term "pseudokinase domain" refers to a portion of a polypeptide or nucleic acid that encodes a portion of the polypeptide, where the portion shows homology to a functional kinase but possesses no catalytic activity. A pseudokinase domain may also be referred to as a "kinase-like domain." An example of a pseudokinase domain is the JAK2 pseudokinase domain, also termed the JH2 domain. The N-terminal part of JAK2 pseudokinase domain (JH2) is a regulatory domain that negatively regulates the activity of JAK2.

The term "kinase domain" refers to a portion of a polypeptide or nucleic acid that encodes a portion of the polypeptide, where the portion is required for kinase activity of the polypeptide (*e.g.*, tyrosine kinase activity).

In some methods of the disclosure, mutations may "affect JAK2 kinase activity." The affected JAK2 kinase activity may include kinase activity that increases, decreases, becomes constitutive, stops completely or affects greater, fewer or different targets. A mutation that affects kinase activity may be present in a kinase domain or in a domain associated with a kinase domain such as the JAK2 pseudokinase domain.

As used herein, the term "including" has the same meaning as the term comprising.

### BRIEF DESCRIPTION OF THE FIGURES

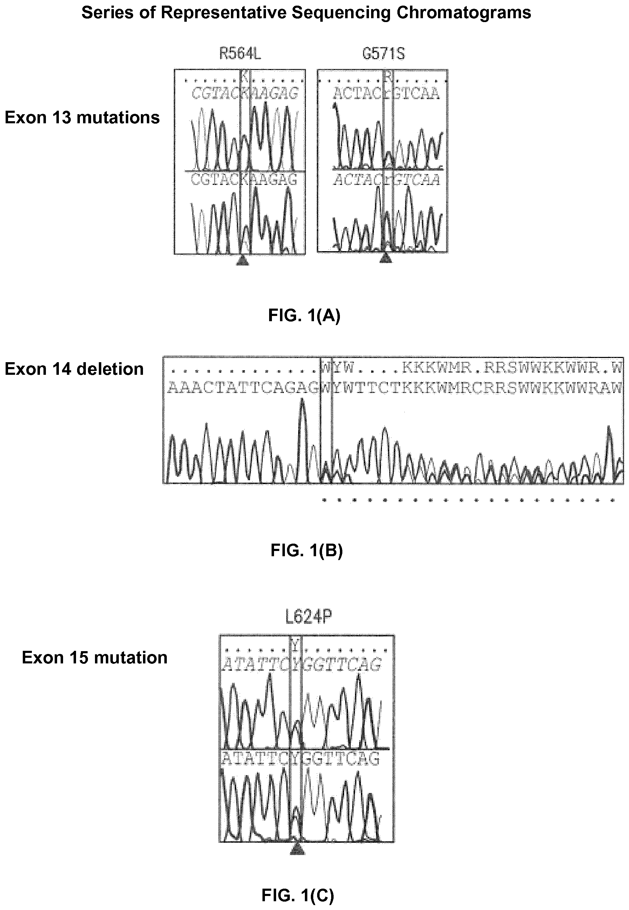

FIG. 1 is a series of representative sequencing chromatograms of Exon 13 mutations (FIG. 1A), Exon 14 deletion (FIG. 1B), and Exon 15 mutation (FIG. 1C). Arrows indicate positions of mutant nucleotides and dots indicate deleted nucleotides.

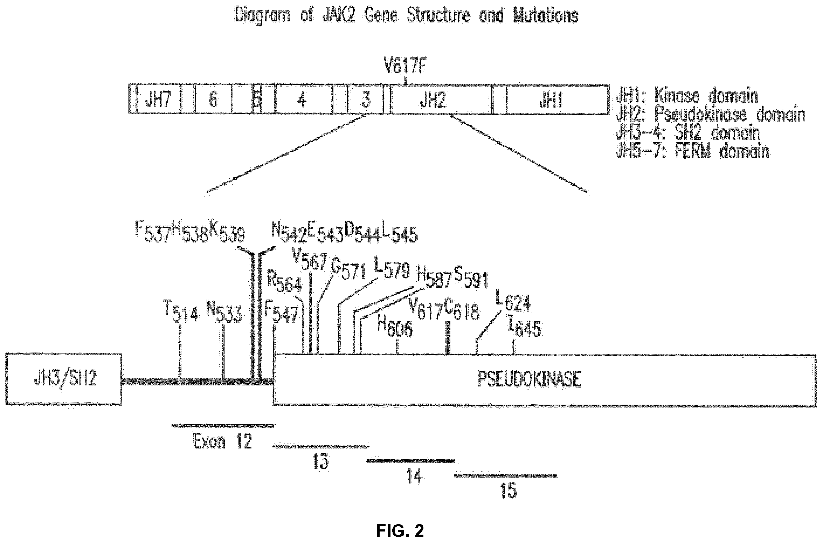

FIG. 2 is a schematic diagram of the JAK2 gene structure with some of the mutated residues in exons 12-15 shown.

### DETAILED DESCRIPTION OF THE INVENTION

The present disclosure is based on the discovery of previously unknown mutations in the JAK2 nucleic acid and protein which have been associated with myeloproliferative diseases. The mutations are shown in Table 2.

### JAK2 nucleic acid

JAK2 genomic nucleic acid is located in human chromosome 9. Exemplary sequences of human JAK2 genomic sequences include but are not limited to GenBank Accession numbers: NG_009904, CM000260, CM000671, GL000069. These sequences are incorporated herein by reference.

Exemplary sequences of all or portions of human JAK2 mRNA include but are not limited to GenBank Accession numbers: NM_004972, human JAK2 mRNA. These sequences are incorporated herein by reference. Exemplary sequence of JAK2 mRNA is provided in SEQ ID NO: 1.

The JAK2 nucleic acids of this invention include, for example, nucleic acids that are substantially identical to a portion of the JAK2 nucleotide sequence of SEQ ID NO: 1 and further comprise one or more of the following mutations listed in Table 2. In some aspects, the JAK2 nucleic acids have at least 10, 12. 14. 16. 18, 20, 25, 30, 40, 50, 75, 100, or more nucleotides in length. In some other aspects, the JAK2 nucleic acids are less than 6000, 5000, 4000, 3000, 2000, 1000, 750, 500, 400, 300, or 200 nucleotides in length.

### Biological Sample Collection and Preparation

The methods and compositions of this disclosure may be used to detect mutations in the JAK2 gene and/or JAK2 protein using a biological sample obtained from an individual. The nucleic acid (DNA or RNA) may be isolated from the sample according to any methods well known to those of skill in the art. If necessary the sample may be collected or concentrated by centrifugation and the like. The cells of the sample may be subjected to lysis, such as by treatments with enzymes, heat, surfactants, ultrasonication, or a combination thereof. The lysis treatment is performed in order to obtain a sufficient amount of nucleic acid derived from the individual's cells to detect using polymerase chain reaction. Alternatively, mutations in the JAK2 gene may be detected using an acellular bodily fluid according to the methods described in U.S. Patent Application US 2007-0248961

### Plasma or Serum Preparation Methods

Methods of plasma and serum preparation are well known in the art. Either "fresh" blood plasma or serum, or frozen (stored) and subsequently thawed plasma or serum may be used. Frozen (stored) plasma or serum should optimally be maintained at storage conditions of -20 to -70 degrees centigrade until thawed and used. "Fresh" plasma or serum should be refrigerated or maintained on ice until used, with nucleic acid (e.g., RNA, DNA or total nucleic acid) extraction being performed as soon as possible. Exemplary methods are described below.

### Nucleic Acid Extraction and Amplification

The nucleic acid to be amplified may be from a biological sample such as an organism, cell culture, tissue sample, and the like. The biological sample can be from a subject which includes any animal, preferably a mammal. A preferred subject is a human, which may be a patient presenting to a medical provider for diagnosis or treatment of a disease. The biological sample may be obtained from a stage of life such as a fetus, young adult, adult, and the like. Particularly preferred subjects are humans being tested for the existence of a JAK2 mutation.

Various methods of extraction are suitable for isolating the DNA or RNA. Suitable methods include phenol and chloroform extraction. See Maniatis et al., Molecular Cloning, A Laboratory Manual, 2d, Cold Spring Harbor Laboratory Press, page 16.54 (1989). Numerous commercial kits also yield suitable DNA and RNA including, but not limited to, QIAamp™ mini blood kit, Agencourt Genfind™, Roche Cobas® Roche MagNA Pure® or phenol:chloroform extraction using Eppendorf Phase Lock Gels®, and the NucliSens extraction kit (Biomerieux, Marcy l'Etoile, France). In other methods, mRNA may be extracted from patient blood/bone marrow samples using MagNA Pure LC mRNA HS kit and Mag NA Pure LC Instrument (Roche Diagnostics Corporation, Roche Applied Science, Indianapolis, IN).

Numerous methods are known in the art for isolating total nucleic acid, DNA and RNA from blood, serum, plasma and bone marrow or other hematopoietic tissues. In fact, numerous published protocols, as well as commercial kits and systems are available. By way of example but not by way of limitation, examples of such kits, systems and published protocols are described below. Commercially available kits include Qiagen products such as the QiaAmp DNA Blood MiniKit (Cat.# 51104, Qiagen, Valencia, CA), the QiaAmp RNA Blood MiniKit (Cat.# 52304, Qiagen, Valencia, CA); Promega products such as the Wizard Genomic DNA Kit (Cat.# A1620, Promega Corp. Madison, WI), Wizard SV Genomic DNA Kit (Cat.# A2360, Promega Corp. Madison, WI), the SV Total RNA Kit (Cat.# X3100, Promega Corp. Madison, WI), PolyATract System (Cat.# Z5420, Promega Corp. Madison, WI), or the PurYield RNA System (Cat.# Z3740, Promega Corp. Madison, WI).

### Extraction of RNA from Plasma or Serum

Plasma RNA is highly sensitive and may replace DNA-based testing because of the relative abundance of the RNA and the ease in detecting deletions such as, for example, deletion of Exon 14. Circulating extracellular deoxyribonucleic acid (DNA), including tumor-derived or associated extracellular DNA, is also present in plasma and serum. *See* Stroun, M., et al., Oncology 46:318-322, (1989). Since this DNA will additionally be extracted to varying degrees during the RNA extraction methods described above, it may be desirable or necessary (depending upon clinical objectives) to further purify the RNA extract and remove trace DNA prior to proceeding to further RNA analysis. This may be accomplished using DNase, for example by the method as described by Rashtchian, A., PCR Methods Applic. 4:S83-S91, (1994), as follows.

Glass beads, Silica particles or Diatom Extraction: RNA may be extracted from plasma or serum using silica particles, glass beads, or diatoms, as in the method or adaptations of Boom, R., et al., J. Clin. Micro. 28:495-503, (1990); Cheung, R. C., et al., J. Clin Micro. 32:2593-2597, (1994).

Acid Guanidinium Thiocyanate-Phenol-Chloroform Extraction: As an alternative method, RNA may be extracted from plasma or serum using the Acid Guanidinium Thiocyanate-Phenol-chloroform extraction method described by Chomczynski, P. and Sacchi, N., Analytical Biochemistry 162:156-159, (1987), as follows.

Alternative Nucleic Acid Extraction Methods: Alternative methods may be used to extract RNA from body fluids including but not limited to centrifugation through a cesium chloride gradient, including the method as described by Chirgwin, J. M., et al., Biochemistry 18:5294-5299, (1979), and co-precipitation of extracellular RNA from plasma or serum with gelatin, such as by adaptations of the method of Fournie, G. J., et al., Analytical Biochemistry 158:250-256, (1986), to RNA extraction.

### Alternative Nucleic Acid Amplification Methods

Alternative methods of nucleic acid amplification which may be used include variations of RT-PCR, including quantitative RT-PCR, for example as adapted to the method described by Wang, A. M. et al., Proc. Natl. Acad. Sci. USA 86:9717-9721, (1989), or by Karet, F. E., et al., Analytical Biochemistry 220:384-390, (1994). Another method of nucleic acid amplification or mutation detection which may be used is ligase chain reaction (LCR), as described by Wiedmann, et al., PCR Methods Appl. 3:551-564, (1994). An alternative method of amplification or mutation detection is allele specific PCR (ASPCR). ASPCR which utilizes matching or mismatching between the template and the 3' end base of a primer well known in the art. See e.g., U.S. Patent 5,639,611.

Another method of mutation detection is nucleic acid sequencing. Sequencing can be performed using any number of methods, kits or systems known in the art. One example is using dye terminator chemistry and an ABI sequencer (Applied Biosystems, Foster City, CA). Sequencing also may involve single base determination methods such as single nucleotide primer extension ("SNapShot®" sequencing method) or allele or mutation specific PCR. The SNaPshot® Multiplex System is a primer extension-based method that enables multiplexing up to 10 SNPs (single nucleotide polymorphisms). The chemistry is based on the dideoxy single-base extension of an unlabeled oligonucleotide primer (or primers). Each primer binds to a complementary template in the presence of fluorescently labeled ddNTPs and AmpliTaq® DNA Polymerase, FS. The polymerase extends the primer by one nucleotide, adding a single ddNTP to its 3' end. SNaPshot® Multiplex System is commercially available (ABI PRISM® SNaPshot® Multiplex kit, Applied Biosystems Foster City, CA). Products generated using the ABI PRISM® SNaPshot® Multiplex kit can be analyzed with GeneScan® Analysis Software version 3.1 or higher using ABI PRISM®310 Genetic Analyzer, ABI PRISM® 3100 Genetic Analyzer or ABI PRISM® 3700 DNA Analyzer.

### Exemplary Methods for Detection of JAK2 DNA and RNA Mutations

Nucleic acid (e.g., total nucleic acid) may be extracted from patient's biological sample using any appropriate method. Next, an RT-PCR reaction may be performed using either the total nucleic acid preparation or the RNA preparation to specifically amplify a portion of the patient RNA. An exemplary one-step RT-PCR system is the Superscript III System (Invitrogen, Carlsbad, CA). Other methods and systems for RT-PCR reactions are well known in the art and are commercially available. By way of example, but not by way of limitation, a primer pair for JAK2 may be 5'-TGT AAA ACG ACG GCC AGT CTA AAT GCT GTC CCC CAA AG-3' (forward primer, SEQ ID NO: 6) and 5'-CAG GAA ACA GCT ATG ACC CCA TGC CAA CTG TTT AGC AA-3' (reverse primer, SEQ ID NO: 7). The RT-PCR product may then be purified, for example by gel purification, and the resulting purified product may be sequenced. Nucleic acid sequencing methods are known in the art; an exemplary sequencing method includes the ABI Prism BigDye Terminator v3.1 Cycle Sequencing Kit (Applied Biosystems, Foster City, CA). The sequencing data may then be analyzed for the presence or absence of one or more mutations in JAK2 nucleic acid. The sequencing data may also be analyzed to determine the proportion of wild-type to mutant nucleic acid present in the sample.

The presence or absence of JAK2 mutations can be determined in a nucleic acid by sequencing appropriate portions of the JAK2 gene containing the mutations sought to be detected. For example, each amplicon of the JAK2 gene is sequenced with forward and reverse primers. In another approach, JAK2 mutations that change susceptibility to digestion by one or more endonuclease restriction enzymes may be used to detect the mutations. In another aspect, the presence of one or more JAK2 mutations can be determined by allele specific amplification. In yet another aspect, the presence of one or more JAK2 mutations can be determined by primer extension. In yet a further aspect, the presence of one or more JAK2 mutations can be determined by oligonucleotide ligation. In another aspect, the presence of one or more JAK2 mutations can be determined by hybridization with a detectably labeled probe containing the mutant JAK2 sequence. The presence or absence of JAK2 mutations can be determined by any known or future method.

### Detection of Mutated JAK2 Proteins

According to the disclosure, the presence or absence of JAK2 mutations can also be determined by analyzing the JAK2 protein (SEQ ID NO: 2) encoded by the mutated JAK2 gene. The mutations include those shown in Table 2. Detection of JAK2 mutations at the protein level can be detected by any method well known in the field. In one aspect, detection of JAK2 mutations is carried out by isolating JAK2 protein and subjecting it to amino acid sequence determination. This may require fragmenting the protein by proteolytic or chemical means prior to sequencing. Methods of determining an amino acid sequence are well known in the art.

Detection of mutated JAK2 proteins can be accomplished using, for example, antibodies, aptamers, ligands/substrates, other proteins or protein fragments, other protein-binding agents, or mass spectrometry analysis of fragments. Preferably, protein detection agents are specific for the mutated JAK2 protein of the present disclosure and can therefore discriminate between a mutated protein and the wild-type protein or another variant form. This can generally be accomplished by, for example, selecting or designing detection agents that bind to the region of a protein that differs between the variant and wild-type protein.

One preferred agent for detecting a mutated JAK2 protein is an antibody capable of selectively binding to a variant form of the protein. Antibodies capable of distinguishing between wild-type and mutated JAK2 protein may be created by any suitable method known in the art. The antibodies may be monoclonal or polyclonal antibodies, single chain or double chain, chimeric or humanized antibodies or portions of immunoglobulin molecules containing the portions known in the state of the art to correspond to the antigen binding fragments.

Methods for manufacturing polyclonal antibodies are well known in the art. Typically, antibodies are created by administering (e.g., via subcutaneous injection) the mutated JAK2 protein immunogenic fragment containing the mutation to white New Zealand rabbits. The JAK2 antigen is typically injected at multiple sites and the injections are repeated multiple times (e.g., approximately bi-weekly) to induce an immune response. Desirably, the rabbits are simultaneously administered an adjuvant to enhance anti-JAK2 immunity. The polyclonal antibodies are then purified from a serum sample, for example, by affinity chromatography using the same JAK2 antigen to capture the antibodies. The antibodies can be made specific to the mutation by removing antibodies cross-reacting with native JAK2.

In vitro methods for detection of the mutated JAK2 proteins also include, for example, enzyme linked immunosorbent assays (ELISAs), radioimmunoassays (RIA), Western blots, immunoprecipitations, immunofluorescence, and protein arrays/chips (e.g., arrays of antibodies or aptamers). For further information regarding immunoassays and related protein detection methods, see Current Protocols in Immunology, John Wiley & Sons, N.Y., and Hage, "Immunoassays", Anal Chem. 1999 Jun. 15; 71(12):294R-304R. Additional analytic methods of detecting amino acid variants include, but are not limited to, altered electrophoretic mobility (e.g., 2-dimensional electrophoresis), altered tryptic peptide digest, altered JAK2 kinase activity in cell-based or cell-free assay, alteration in ligand or antibody-binding pattern, altered isoelectric point, and direct amino acid sequencing.

### Diagnostic Tools

JAK2 nucleic acid may be used as tools to diagnose an individual as having (or as likely to develop) a myeloproliferative disease. Alternatively, the JAK2 mutation status, used alone or in combination with other clinical parameters, also may be used to determine a prognosis for a patient diagnosed as having a myeloproliferative disease. Exemplary mutations in JAK2 nucleic acid is listed in Tables 1 and 2.

In some aspects, the JAK2 nucleic acids further comprise a detectable label and are used as a probe ("JAK2 probe") to detect mutated JAK2 nucleic acids in a patient sample. The JAK2 probe may be detectably labeled by methods known in the art. Useful labels include, for example, fluorescent dyes (e.g., Cy5®, Cy3®, FITC, rhodamine, lanthamide phosphors, Texas red, FAM, JOE, Cal Fluor Red 610®, Quasar 670®), radioisotopes (e.g., ³²P, ³⁵S, ³H, ¹⁴C , ¹²⁵I, ¹³¹I), electron-dense reagents (e.g., gold), enzymes (e.g., horseradish peroxidase, beta-galactosidase, luciferase, alkaline phosphatase), colorimetric labels (e.g., colloidal gold), magnetic labels (e.g., Dynabeads™), biotin, dioxigenin, or haptens and proteins for which antisera or monoclonal antibodies are available. Other labels include ligands or oligonucleotides capable of forming a complex with the corresponding receptor or oligonucleotide complement, respectively. The label can be directly incorporated into the nucleic acid to be detected, or it can be attached to a probe (e.g., an oligonucleotide) or antibody that hybridizes or binds to the nucleic acid to be detected.

In other aspects, the JAK2 probes are TaqMan® probes, molecular beacons, and Scorpions (e.g., Scorpion™ probes). These types of probes are based on the principle of fluorescence quenching and involve a donor fluorophore and a quenching moiety. The term "fluorophore" as used herein refers to a molecule that absorbs light at a particular wavelength (excitation frequency) and subsequently emits light of a longer wavelength (emission frequency). The term "donor fluorophore" as used herein means a fluorophore that, when in close proximity to a quencher moiety, donates or transfers emission energy to the quencher. As a result of donating energy to the quencher moiety, the donor fluorophore will itself emit less light at a particular emission frequency that it would have in the absence of a closely positioned quencher moiety.

The term "quencher moiety" as used herein means a molecule that, in close proximity to a donor fluorophore, takes up emission energy generated by the donor and either dissipates the energy as heat or emits light of a longer wavelength than the emission wavelength of the donor. In the latter case, the quencher is considered to be an acceptor fluorophore. The quenching moiety can act via proximal (i.e., collisional) quenching or by Forster or fluorescence resonance energy transfer ("FRET"). Quenching by FRET is generally used in TaqMan® probes while proximal quenching is used in molecular beacon and Scorpion™ type probes. Suitable quenchers are selected based on the fluorescence spectrum of the particular fluorophore. Useful quenchers include, for example, the Black Hole™ quenchers BHQ-1, BHQ-2, and BHQ-3 (Biosearch Technologies, Inc.), and the ATTO-series of quenchers (ATTO 540Q, ATTO 580Q, and ATTO 612Q; Atto-Tec GmbH).

With Scorpion primers, sequence-specific priming and PCR product detection is achieved using a single molecule. The Scorpion primer maintains a stem-loop configuration in the unhybridized state. The fluorophore is attached to the 5' end and is quenched by a moiety coupled to the 3' end, although in suitable aspects, this arrangement may be switched The 3' portion of the stem also contains sequence that is complementary to the extension product of the primer. This sequence is linked to the 5' end of a specific primer via a non-amplifiable monomer. After extension of the primer moiety, the specific probe sequence is able to bind to its complement within the extended amplicon thus opening up the hairpin loop. This prevents the fluorescence from being quenched and a signal is observed. A specific target is amplified by the reverse primer and the primer portion of the Scorpion primer, resulting in an extension product. A fluorescent signal is generated due to the separation of the fluorophore from the quencher resulting from the binding of the probe element (e.g., the JAK2 probe) of the Scorpion primer to the extension product.

TaqMan® probes (Heid, et al., Genome Res 6: 986-994, 1996) use the fluorogenic 5' exonuclease activity of Taq polymerase to measure the amount of target sequences in cDNA samples. TaqMan® probes are oligonucleotides that contain a donor fluorophore usually at or near the 5' base, and a quenching moiety typically at or near the 3' base. The quencher moiety may be a dye such as TAMRA or may be a non-fluorescent molecule such as 4-(4 -dimethylaminophenylazo) benzoic acid (DABCYL). See Tyagi, et al., 16 Nature Biotechnology 49-53 (1998). When irradiated, the excited fluorescent donor transfers energy to the nearby quenching moiety by FRET rather than fluorescing. Thus, the close proximity of the donor and quencher prevents emission of donor fluorescence while the probe is intact.

TaqMan® probes are designed to anneal to an internal region of a PCR product. When the polymerase (e.g., reverse transcriptase) replicates a template on which a TaqMan® probe is bound, its 5' exonuclease activity cleaves the probe. This ends the activity of the quencher (no FRET) and the donor fluorophore starts to emit fluorescence which increases in each cycle proportional to the rate of probe cleavage. Accumulation of PCR product is detected by monitoring the increase in fluorescence of the reporter dye (note that primers are not labeled). If the quencher is an acceptor fluorophore, then accumulation of PCR product can be detected by monitoring the decrease in fluorescence of the acceptor fluorophore.

The JAK2 polypeptides of this disclosure include, for example, polypeptides that are substantially identical to a portion of the JAK2 amino acid sequence of SEQ ID NO: 2 and further comprise one or more of the following mutations listed in Table 2. These polypeptides may be used as tools to diagnose an individual as having (or as likely to develop) a myeloproliferative disease. Alternatively, the JAK2 mutation status, used alone or in combination with other clinical parameters, also may be used to determine a prognosis for a patient diagnosed as having a myeloproliferative disease. In some aspects, the JAK2 polypeptides have at least 10, 12, 14, 16, 18, 20, 25, 30, 40, 50, 75, 100, or more amino acids. In some aspects, the JAK2 polypeptide is less than 1100, 1000, 900, 800, 700, 600, 500, or 400 amino acids.

### Kits For Detecting JAK2 Mutations

The disclosure also provides kits for detecting JAK2 mutations. The kits will contain at least a primer pair capable of amplifying a target JAK2 nucleic acid sequence of SEQ ID NO: 1 and a means for detecting a JAK2 mutation in the target. Preferably, the amplification using the primer pair of the kit results in a reaction product having at least 20, 40, 60, 80, 100, 125, 150, 200, 300, 500, or more nucleotides. Suitable primer pairs include, for example, primers having the sequence of SEQ ID NOs: 6 and 7. Suitable means for detecting a JAK2 mutation in the reaction product make use of a detectably labeled JAK2 probe, such as those described herein.

### Diagnosis, Detection, and Prognosis

The presence of the JAK2 mutations, such as, for example, the mutations listed in Table 2 alone, in combination with each other, or in combination with other JAK2 mutations can be as an indicator of disease.

Without wishing to be bound by any theory, it is believed that the JAK2 mutations allow the activation loop of the kinase domain to move away from the pseudokinase domain, thus leading to constitutive activation of the JAK2-STAT pathway. Mutations in the pseudokinase domain are expected to inactivate the auto-inhibitory function of the pseudokinase domain on the kinase activity. A considerable portion of activating mutations in tyrosine kinases associated with human cancer are loss-of-function alleles residing in the auto-inhibitory domains, for example, point mutations and deletions that result in constitutive tyrosine kinase activation. Therefore, testing for JAK2 mutations should include most of the pseudokinase domain.

Without wishing to be bound by any theory, it has been suggested from homology modeling approaches that residues 537 through 543 (mutation hot spots in exon 12) lie within a loop region bridging the SH2 and JH2 domains of JAK2. In the predicted model, positive polar or hydrophobic interactions between D407-K655, S411-E653, K415-E685 and F408-H608 from the SH2 and the JH2 domains, respectively, are closely packed in the predicted interface that further supported the JH2-JH1 interaction (21). Therefore, mutations in this loop area may disrupt JH2-JH1 interaction leading to constitutive kinase activation, e.g., del/F537-K539ins/L, del/N542-E543, H538QK539L and K539L, all displayed increased JAK2 activation, cytokine-independent hypersensitive proliferation, and in the case of K539L, developed a myeloproliferative phenotype. See *e*.*g*., Scott et al., N Engl J Med 356:459-468 (2007).

One or more of the following determinations may be used to diagnose a patient: determining the presence or absence of a specific JAK2 mutation, determining the zygosity status of the sample, and determining the ratio of mutant to wild-type JAK2 nucleic acid or mRNA in the sample. For example, patients found to carry a specific JAK2 mutation by the methods of the disclosure may be recommended for further testing to verify an MPD diagnosis, or detection of the mutation may be used to finally confirm a preliminary diagnosis of MPD (*e*.*g*., if a patient is symptomatic for an MPD and also tests positive for a specific JAK2 mutation known to be indicative of a particular MPD, the patient may be finally diagnosed with an MPD such as PV). Similarly, methods of the disclosure may be used to diagnose patients who are asymptomatic for MPD, for example patients who are in the very early stages of an MPD. JAK2 mutations may also be detected in MPD patients who are undergoing treatment; if the ratio of mutant to wild-type JAK2 nucleic acid or the zygosity status of the sample changes during treatment, a different diagnosis may be made.

One or more of the following determinations may be used to treat a patient: determining the presence or absence of a specific JAK2 mutation, determining the zygosity status of the sample, and determining the ratio of mutant to wild-type JAK2 nucleic acid in a sample. A physician or treatment specialist may administer, forego or alter a treatment or treatment regime based on one or more of the determinations. Further, the number of cancerous cells carrying the mutation may change during the course of an MPD and monitoring the ratio, the zygosity status, and/or the presence or absence of a JAK2 mutation may be an indication of disease status or treatment efficacy. For example, treatment may reduce the number of mutant cancerous cells, or the disease may become worse with time, and the number of diseased cells may increase. Additionally, one or more of the determinations may aid in patient prognosis and quality of life decisions. For example, decisions about whether to continue - or for how long to continue - a painful, debilitating treatment such as chemotherapy may be made.

The zygosity status and the ratio of wild-type to mutant nucleic acid in a sample may be determined by methods known in the art including sequence-specific, quantitative detection methods. Other methods may involve determining the area under the curves of the sequencing peaks from standard sequencing electropherograms, such as those created using ABI Sequencing Systems, (Applied Biosystems, Foster City CA). For example, the presence of only a single peak such as a "G" on an electropherogram in a position representative of a particular nucleotide is an indication that the nucleic acids in the sample contain only one nucleotide at that position, the "G." The sample may then be categorized as homozygous because only one allele is detected. The presence of two peaks, for example, a "G" peak and a "T" peak in the same position on the electropherogram indicates that the sample contains two species of nucleic acids; one species carries the "G" at the nucleotide position in question, the other carries the "T" at the nucleotide position in question. The sample may then be categorized as heterozygous because more than one allele is detected.

The sizes of the two peaks may be determined (*e*.*g*., by determining the area under each curve), and a ratio of the two different nucleic acid species may be calculated. A ratio of wild-type to mutant nucleic acid may be used to monitor disease progression, determine treatment, or to make a diagnosis. For example, the number of cancerous cells carrying a specific JAK2 mutation may change during the course of an MPD. If a base line ratio is established early in the disease, a later determined higher ratio of mutant nucleic acid relative to wild-type nucleic acid may be an indication that the disease is becoming worse or a treatment is ineffective; the number of cells carrying the mutation may be increasing in the patient. A lower ratio of mutant relative to wild-type nucleic acid may be an indication that a treatment is working or that the disease is not progressing; the number of cells carrying the mutation may be decreasing in the patient.

### EXAMPLES

### EXAMPLE 1: Detection of the JAK2 Mutations

Approximately 20,000 patient samples with suspicious diagnosis of MPD over a period of 7 months (from November, 2007 through June, 2008) were collected and screened for *JAK2* gene mutation through the region of entire exons 12-15. Five milliliters of blood in Lavender (purple top tubes) containing ethylenediaminetetraacetic acid (EDTA) anticoagulant were required for analysis. Blood samples were refrigerated (not frozen) or kept at room temperature no more than 48 hr if immediate nucleic acid extraction was not possible.

Total nucleic acids were isolated from peripheral blood plasma by the NucliSens extraction kit (bioMerieux Inc., Durham, NC, USA) according to the manufacturer's instructions. First strand cDNAs were then prepared by reverse transcription of total RNAs with random primers at 55°C for 30 min, followed by PCR reactions using SuperSript III one-step RT-PCR system with Platinum Taq DNA polymerase (Invitrogen, Carlsbad, CA, USA). For amplification of the *JAK2* exons 12-15, the following primer set and conditions were used: 5'-TGT AAA ACG ACG GCC AGT CTA AAT GCT GTC CCC CAA AG-3' (forward primer, SEQ ID NO: 6) and 5'-CAG GAA ACA GCT ATG ACC CCA TGC CAA CTG TTT AGC AA-3' (reverse primer, SEQ ID NO: 7); initial step of 2 min at 94 °C, followed by 40 cycles of 94 °C for 15 sec, 60 °C for 30 sec, and 68 °C for 1 min, and ending with one step of 68 °C for 7 min. The 491-bp amplified products were filter-purified by Multiscreen PCR plates (Millipore, Billerica, MA, USA) and sequenced in both directions using the ABI Prism BigDye® Terminator v3.1 Cycle Sequencing Kit and detected by ABI PRISM 3100 Genetic Analyzer (Applied Biosystems, Foster City, CA, USA). Sequence data was then base-called, assembled and analyzed by ABI Prism® SeqScape software (Applied Biosystems, Foster City, CA, USA) using the *JAK2* sequence (accession number NM004972) as a reference.

Table 1 presents a mutation analysis of JAK2 gene exon 12 (SEQ ID NO: 3), exon 13 (SEQ ID NO: 4), exon 14 (SEQ ID NO: 5), and exon 15 from a large group of suspected MPD blood samples.

**Table 1: Mutation Analysis**

| **Exon*** | **Mutation** | **Codon change** | **No. of cases** | **Pattern on sequencing#** |
|---|---|---|---|---|
| 12 | Silent | H531 | 1 | Heterozygous |
| 12 | Duplication | dupl/V536-F547 | 2 | Heterozygous |
| 12 | Deletion | del H538 | 1 | Heterozygous |
| 12 | Deletion | del/H538-K539 | 1 | Heterozygous |
| 12 | Deletion | del/N542-E543 | 5 | Heterozygous |
| 12 | Deletion | del/E543-D544 | 3 | Heterozygous |
| | Indels | del/F537-K539 | 1 | |
| 12 | | ins/K | | Heterozygous |
| | Indels | del/F537-K539 | 2 | |
| 12 | | ins/L | | Heterozygous |
| | Indels | del/H538-K539 | 4 | |
| 12 | | ins/L | | Heterozygous |
| | Indels | del/R541-E543 | 1 | |
| 12 | | ins/K | | Heterozygous |
| | Indels | del/I540-D544 | 1 | |
| 12 | | ins/MK | | Heterozygous |
| | Indels | del/N542-D544 | 1 | |
| 12 | | ins/N | | Heterozygous |
| 12 | Missense | T514M | 2 | Heterozygous |
| 12 | Missense | N533Y | 1 | Heterozygous |
| 12 | Missense | K539L | 2 | Heterozygous |
| | Missense | H538Q | 1 | |
| 12 | | K539L | | Heterozygous |
| | Missense | K539L | 1 | |
| 12 | | L545V | | Heterozygous |
| 12 | Missense | F547L | 1 | Heterozygous |
| 13 | Silent | G562 | 2 | Heterozygous |
| 13 | Silent | Y570 | 4 | Heterozygous |
| 13 | Silent | F556 | 1 | Heterozygous |
| 13 | Missense | R564L | 3 | Heterozygous |
| 13 | Missense | R564Q | 2 | Heterozygous |
| 13 | Missense | V567A | 1 | Heterozygous |
| 13 | Missense | G571S | 3 | Heterozygous |
| 13 | Missense | G571R | 1 | Heterozygous |
| 13 | Missense | L579F | 1 | Heterozygous |
| 13 | Missense | H587N | 1 | Heterozygous |
| 13 | Missense | S591L | 1 | Heterozygous |
| 14 | Deletion | S593-N622 | 2 | Heterozygous |
| 14 | Missense | H606Q | 1 | Heterozygous |
| 14 | Missense | V617F | >2000 | Hetero/Homo |
| 14 | Missense | V617I | 1 | Heterozygous |
| | Missense | V617F | | |
| 14 | | C618R | 2 | Hetero/Homo |
| 14 | Missense | C618R | 1 | Heterozygous |
| 15 | Missense | L624P | 1 | Heterozygous |
| 15 | Missense | I645V | 1 | Heterozygous |

| | | | | |
|---|---|---|---|---|
| **Exon 12 (residues 511-547); Exon13 (residues 548-592); Exon14 (residues 593-622)*. | | | | |

### EXAMPLE 2: Additional JAK2 Mutations

Table 2 presents additional specific examples of novel JAK2 mutations identified by sequencing, along with their deduced amino acid substitutions.

**Table 2: Exons 12, 13, 14, and 15 Mutations**

| **Exon 12** | |
|---|---|
| JAK2 Mutation Nucleic Acid; (Codon Change) | JAK2 Mutation Protein; (Amino Acid Change) |
| Missense Mutations | |
| c2035t; (acg>atg) | T514M; (Thr → Met) |
| a2091t ; (aac>tac) | N533Y (Asn → Tyr) |
| t2127c; (ttg>gtg) | L545V; (Leu → Val) |
| t2133c; (ttt>ctt) | F547L; (Phe → Leu) |
| del: deletion; ins: insertion | |

| **Exon 13** | |
|---|---|
| JAK2 Mutation Nucleic Acid; (Codon Change) | JAK2 Mutation Protein; (Amino Acid Change) |
| Missense Mutations | |
| t2160g ttt>gtt | F556V (Phe → Val) |
| c2180t; (ggc>ggt) | G562 silent; (Gly) |
| g2185t; (cga>cta) | R564L; (Arg → Leu) |
| g2185a; (cga>caa) | R564Q; (Arg → Gln) |
| g2193t; (gta>tta) | V567L; (Val → Leu) |
| t2194c; (gta>gca) | V567A; (Val → Ala) |
| c2204t; (tac>tat) | Y570 Silent; (Tyr) |
| g2205a; (ggt>agt) | G571S; (Gly → Ser) |
| g2205c; (ggt>cgt) | G571R; (Gly → Arg) |
| c2229t; (ctt>ttt) | L579F; (Leu → Phe) |
| c2253a; (cac>aac) | H587N; (His → Asn) |
| c2266t; (tca>tta) | S591L; (Ser → Leu) |

| **Exon 14** | |
|---|---|
| JAK2 Mutation Nucleic Acid; (Codon Change) | JAK2 Mutation Protein; (Amino Acid Change) |
| Exon 14 Missense and Deletion Mutations | |
| c2312a; (cac>caa) | H606Q; (His → Gln) |
| 2271-2358 (exon 14 deletion) | S593-N622 |

| **Exon 15** | |
|---|---|
| Exon 15 Missense Mutations | |
| t2365c; (ctg>ccg) | L624P; (Leu → Pro) |
| a2427g; (ata>gta) | I645V; (Ile → Val) |

Unless otherwise defined, all technical and scientific terms used herein have the same meaning as commonly understood by one of ordinary skill in the art to which this invention belongs.

## Claims

1. A method for detecting a hematopoietic disease comprising detecting the presence or absence of a JAK2 mutation using a biological sample obtained from an individual, comprising:
a) evaluating a sample from the individual for the presence or absence of a mutation in JAK2 nucleic acid or JAK2 polypeptide, wherein said mutation in the JAK2 nucleic acid is t2160g, and the mutation in the JAK2 polypeptide is the amino acid substitution F556V; and
b) identifying said individual as having a hematopoietic disease when said JAK2 nucleic acid or JAK2 polypeptide, respectively, comprises said mutation.

2. The method of claim 1, wherein the JAK2 nucleic acid is RNA.

3. The method of claims 1 or 2, wherein the individual is a patient and wherein said biological sample is from a biological fluid from the patient.

4. The method of any one of claims 1-3, wherein said biological sample is selected from the group consisting of blood, serum, and plasma.

5. The method of any one of claims 1-4, wherein said JAK2 nucleic acid or said JAK2 polypeptide further comprises at least one additional mutation shown in Table 2.

6. The method of any one of claims 1-5, wherein said hematopoietic disease is a myeloproliferative disease.

7. The method of claim 6, wherein said myeloproliferative disease is selected from the group consisting of polycythemia vera, essential thrombocythemia, idiopathic myelofibrosis, and unclassified myeloproliferative disease.

8. The method of any one of claims 1-7, wherein said evaluating step comprises amplifying JAK2 nucleic acid and hybridizing the amplified nucleic acid with a detection oligonucleotide that is capable of specifically detecting JAK2 nucleic acid under hybridization conditions.

9. The method of any one of claims 1-8, wherein said evaluating step comprises amplifying JAK2 nucleic acid and performing direct sequencing analysis of the amplified nucleic acid.

10. The method of any one of claims 1-9, wherein said evaluating step comprises using an antibody that specifically binds to the mutated JAK2 polypeptide.

11. An isolated nucleic acid comprising at least 17 contiguous nucleotides corresponding to SEQ ID NO:1, wherein said isolated nucleic acid comprises the mutation t2160g, wherein said nucleic acid is less than 5000 nucleotides in length.

12. The isolated nucleic acid of claim 11, wherein said nucleic acid further comprises at least one additional mutation shown in Table 2, and/or wherein said nucleic acid is labeled with a detectable label.

13. An isolated polypeptide comprising at least 10 contiguous amino acids corresponding to SEQ ID NO: 2, wherein said isolated polypeptide comprises the amino acid substitution F556V, wherein said polypeptide is less than 1100 amino acids.

14. The isolated polypeptide of claim 13, wherein said polypeptide further comprises at least one additional mutation shown in Table 2, and/or wherein said polypeptide is labeled with a detectable label.

## Patentansprüche

1. Verfahren zum Detektieren einer hämatopoetischen Krankheit, umfassend Detektieren der Präsenz oder Absenz einer JAK2-Mutation unter Verwendung einer von einem Individuum erhaltenen biologischen Probe, umfassend:
a) Untersuchen einer Probe vom Individuum auf Präsenz oder Absenz einer Mutation in JAK2-Nukleinsäure oder JAK2-Polypeptid, wobei die Mutation in der JAK2-Nukleinsäure t2160g ist, und die Mutation im JAK2-Polypeptid die Aminosäuresubstitution F556V ist; und
b) Identifizieren des Individuums als eine hämatopoetischen Krankheit habend, wenn die JAK2-Nukleinsäure oder das JAK2-Polypeptid jeweils die Mutation umfassen.

2. Verfahren gemäß Anspruch 1, wobei die JAK2-Nukleinsäure RNA ist.

3. Verfahren gemäß Anspruch 1 oder 2, wobei das Individuum ein Patient ist, und wobei die biologische Probe von einer biologischen Flüssigkeit des Patienten stammt.

4. Verfahren gemäß irgendeinem der Ansprüche 1-3, wobei die biologische Probe ausgewählt wird aus der Gruppe bestehend aus Blut, Serum und Plasma.

5. Verfahren gemäß irgendeinem der Ansprüche 1-4, wobei die JAK2-Nukleinsäure oder das JAK2-Polypeptid weitergehend mindestens eine wie in Tabelle 2 gezeigte Mutation umfasst.

6. Verfahren gemäß irgendeinem der Ansprüche 1-5, wobei die hämatopoetische Krankheit eine myeloproliferative Krankheit ist.

7. Verfahren gemäß Anspruch 6, wobei die myeloproliferative Krankheit ausgewählt wird aus der Gruppe bestehend aus Polycythemia vera, essentieller Thrombozythämie, idiopathischer Myelofibrose, und unklassifizerter myeloproliferativer Krankheit.

8. Verfahren gemäß irgendeinem der Ansprüche 1-7, wobei der Schritt des Untersuchens Amplifizieren von JAK2-Nukleinsäure und Hybridisieren von der amplifizierten Nukleinsäure mit einem Detektions-Oligonukleotid, welches die Fähigkeit hat, JAK2-Nukleinsäure unter Hybridisierungsbedingungen spezifisch zu erkennen, umfasst.

9. Verfahren gemäß irgendeinem der Ansprüche 1-8, wobei der Schritt des Untersuchens Amplifizieren von JAK2-Nukleinsäure und Durchführen von Direkt-Sequenzanalysis der amplifizierten Nukleinsäure umfasst.

10. Verfahren gemäß irgendeinem der Ansprüche 1-9, wobei der Schritt des Untersuchens Verwenden eines Antikörpers, welcher spezifisch an das mutierten JAK2-Polypeptid bindet, umfasst.

11. Isolierte Nukleinsäure, umfassend mindestens 17 aufeinanderfolgende Nukleotide entsprechend SEQ ID NO: 1, wobei die isolierte Nukleinsäure die Mutation t2160g umfasst, wobei die Nukleinsäure weniger als 5000 Nukleotide lang ist.

12. Isolierte Nukleinsäure gemäß Anspruch 11, wobei die Nukleinsäure weitergehend mindestens eine zusätzliche wie in Tabelle 2 gezeigte Mutation umfasst, und/oder wobei die Nukleinsäure mit einem detektierbaren Label markiert ist.

13. Isoliertes Polypeptid, umfassend mindestens 10 aufeinanderfolgende Aminosäuren entsprechend SEQ ID NO: 2, wobei das isolierte Polypeptid die Aminosäuresubstitution F556V umfasst, wobei das Polypeptid weniger als 1100 Aminosäuren hat.

14. Isoliertes Polypeptid gemäß Anspruch 13, wobei das Polypeptid weitergehend mindestens eine zusätzliche wie in Tabelle 2 gezeigte Mutation umfasst, und/oder wobei das Polypeptid mit einem detektierbaren Label markiert ist.

## Revendications

1. Méthode de détection d'une maladie hématopoïétique comprenant la détection de la présence ou de l'absence d'une mutation JAK2 en utilisant un échantillon biologique obtenu d'un individu, comprenant les étapes qui consistent à :
a) évaluer un échantillon provenant de l'individu pour la présence ou l'absence d'une mutation dans l'acide nucléique JAK2 ou le polypeptide JAK2, dans lequel ladite mutation dans l'acide nucléique JAK2 est t2160g, et la mutation dans le polypeptide JAK2 est la substitution d'acide aminé F556V ; et
b) identifier que ledit individu est atteint d'une maladie hématopoïétique quand ledit acide nucléique JAK2 ou le polypeptide JAK2, respectivement, comprend ladite mutation.

2. Méthode selon la revendication 1, dans lequel l'acide nucléique JAK2 est l'ARN.

3. Méthode selon la revendication 1 ou 2, dans laquelle l'individu est un patient et dans laquelle ledit échantillon biologique provient d'un fluide biologique du patient.

4. Méthode selon l'une quelconque des revendications 1 à 3, dans laquelle ledit échantillon biologique est sélectionné parmi le groupe constitué par le sang, le sérum, et le plasma.

5. Méthode selon l'une quelconque des revendications 1 à 4, dans laquelle ledit acide nucléique JAK2 ou ledit polypeptide JAK2 comprend en outre au moins une mutation supplémentaire représentée dans le Tableau 2.

6. Méthode selon l'une quelconque des revendications 1 à 5, dans laquelle ladite maladie hématopoïétique est une maladie myéloproliférative.

7. Méthode selon la revendication 6, dans laquelle ladite maladie myéloproliférative est sélectionnée parmi le groupe constitué par la polyglobulie vraie, la thrombocytémie essentielle, la myélofibrose idiopathique, et une maladie myéloproliférative non classifiée.

8. Méthode selon l'une quelconque des revendications 1 à 7, dans laquelle ladite étape d'évaluation comprend l'amplification de l'acide nucléique JAK2 et l'hybridation de l'acide nucléique amplifié à un oligonucléotide de détection qui est capable de détecter spécifiquement l'acide nucléique JAK2 sous des conditions d'hybridation.

9. Méthode selon l'une quelconque des revendications 1 à 8, dans laquelle ladite étape d'évaluation comprend l'amplification de l'acide nucléique JAK2 et la réalisation d'une analyse de séquençage direct de l'acide nucléique amplifié.

10. Méthode selon l'une quelconque des revendications 1 à 9, dans laquelle ladite étape d'évaluation consiste à utiliser un anticorps qui se lie spécifiquement au polypeptide JAK2 muté.

11. Acide nucléique isolé comprenant au moins 17 nucléotides contigus correspondant à SEQ ID NO : 1, dans lequel ledit acide nucléique isolé comprend la mutation t2160g, dans lequel ledit acide nucléique a une longueur de moins de 5000 nucléotides.

12. Acide nucléique isolé selon la revendication 11, dans lequel ledit acide nucléique comprend en outre au moins une mutation supplémentaire représentée dans le Tableau 2, et/ou dans lequel ledit acide nucléique est marqué avec un marqueur détectable.

13. Polypeptide isolé comprenant au moins 10 acides aminés contigus correspondant à SEQ ID NO : 2, dans lequel ledit polypeptide isolé comprend la substitution d'acide aminé F556V, dans lequel ledit polypeptide compte moins de 1100 acides aminés.

14. Polypeptide isolé selon la revendication 13, dans lequel ledit polypeptide comprend en outre au moins une mutation supplémentaire représentée dans le Tableau 2, et/ou dans lequel ledit polypeptide est marqué avec un marqueur détectable.


### SEQUENCE LISTING

<110> Quest Diagnostics Investments IncorporatedG
<120> COMPOSITIONS AND METHODS FOR DETECTING MUTATIONS IN JAK2 NUCLEIC ACID
<130> QUE14162PCTEPD2
<140> 12/503,318
   <141> 2009-07-15
<150> 61/110,501
   <151> 2008-10-31
<160> 11
<170> PatentIn version 3.5
<210> 1
   <211> 5097
   <212> DNA
   <213> Homo sapiens
<400> 1
   ctgcaggaag gagagaggaa gaggagcaga agggggcagc agcggacgcc gctaacggcc      60
   tccctcggcg ctgacaggct gggccggcgc ccggctcgct tgggtgttcg cgtcgccact     120
   tcggcttctc ggccggtcgg gcccctcggc ccgggcttgc ggcgcgcgtc ggggctgagg     180
   gctgctgcgg cgcagggaga ggcctggtcc tcgctgccga gggatgtgag tgggagctga     240
   gcccacactg gagggccccc gagggcccag cctggaggtc gttcagagcc gtgcccgccc     300
   cggggcttcg cagaccttga cccgccgggt aggagccgcc cctgcgggct cgagggcgcg     360
   ctctggtcgc ccgatctgtg tagccggttt cagaagcagg caacaggaac aagatgtgaa     420
   ctgtttctct tctgcagaaa aagaggctct tcctcctcct cccgcgacgg caaatgttct     480
   gaaaaagact ctgcatggga atggcctgcc ttacgatgac agaaatggag ggaacatcca     540
   cctcttctat atatcagaat ggtgatattt ctggaaatgc caattctatg aagcaaatag     600
   atccagttct tcaggtgtat ctttaccatt cccttgggaa atctgaggca gattatctga     660
   cctttccatc tggggagtat gttgcagaag aaatctgtat tgctgcttct aaagcttgtg     720
   gtatcacacc tgtgtatcat aatatgtttg ctttaatgag tgaaacagaa aggatctggt     780
   atccacccaa ccatgtcttc catatagatg agtcaaccag gcataatgta ctctacagaa     840
   taagatttta ctttcctcgt tggtattgca gtggcagcaa cagagcctat cggcatggaa     900
   tatctcgagg tgctgaagct cctcttcttg atgactttgt catgtcttac ctctttgctc     960
   agtggcggca tgattttgtg cacggatgga taaaagtacc tgtgactcat gaaacacagg    1020
   aagaatgtct tgggatggca gtgttagata tgatgagaat agccaaagaa aacgatcaaa    1080
   ccccactggc catctataac tctatcagct acaagacatt cttaccaaaa tgtattcgag    1140
   caaagatcca agactatcat attttgacaa ggaagcgaat aaggtacaga tttcgcagat     1200
   ttattcagca attcagccaa tgcaaagcca ctgccagaaa cttgaaactt aagtatctta     1260
   taaatctgga aactctgcag tctgccttct acacagagaa atttgaagta aaagaacctg     1320
   gaagtggtcc ttcaggtgag gagatttttg caaccattat aataactgga aacggtggaa     1380
   ttcagtggtc aagagggaaa cataaagaaa gtgagacact gacagaacag gatttacagt     1440
   tatattgcga ttttcctaat attattgatg tcagtattaa gcaagcaaac caagagggtt     1500
   caaatgaaag ccgagttgta actatccata agcaagatgg taaaaatctg gaaattgaac     1560
   ttagctcatt aagggaagct ttgtctttcg tgtcattaat tgatggatat tatagattaa     1620
   ctgcagatgc acatcattac ctctgtaaag aagtagcacc tccagccgtg cttgaaaata     1680
   tacaaagcaa ctgtcatggc ccaatttcga tggattttgc cattagtaaa ctgaagaaag     1740
   caggtaatca gactggactg tatgtacttc gatgcagtcc taaggacttt aataaatatt     1800
   ttttgacttt tgctgtcgag cgagaaaatg tcattgaata taaacactgt ttgattacaa     1860
   aaaatgagaa tgaagagtac aacctcagtg ggacaaagaa gaacttcagc agtcttaaag     1920
   atcttttgaa ttgttaccag atggaaactg ttcgctcaga caatataatt ttccagttta     1980
   ctaaatgctg tcccccaaag ccaaaagata aatcaaacct tctagtcttc agaacgaatg     2040
   gtgtttctga tgtaccaacc tcaccaacat tacagaggcc tactcatatg aaccaaatgg     2100
   tgtttcacaa aatcagaaat gaagatttga tatttaatga aagccttggc caaggcactt     2160
   ttacaaagat ttttaaaggc gtacgaagag aagtaggaga ctacggtcaa ctgcatgaaa     2220
   cagaagttct tttaaaagtt ctggataaag cacacagaaa ctattcagag tctttctttg     2280
   aagcagcaag tatgatgagc aagctttctc acaagcattt ggttttaaat tatggagtat     2340
   gtgtctgtgg agacgagaat attctggttc aggagtttgt aaaatttgga tcactagata     2400
   catatctgaa aaagaataaa aattgtataa atatattatg gaaacttgaa gttgctaaac     2460
   agttggcatg ggccatgcat tttctagaag aaaacaccct tattcatggg aatgtatgtg     2520
   ccaaaaatat tctgcttatc agagaagaag acaggaagac aggaaatcct cctttcatca     2580
   aacttagtga tcctggcatt agtattacag ttttgccaaa ggacattctt caggagagaa     2640
   taccatgggt accacctgaa tgcattgaaa atcctaaaaa tttaaatttg gcaacagaca     2700
   aatggagttt tggtaccact ttgtgggaaa tctgcagtgg aggagataaa cctctaagtg     2760
   ctctggattc tcaaagaaag ctacaatttt atgaagatag gcatcagctt cctgcaccaa     2820
   agtgggcaga attagcaaac cttataaata attgtatgga ttatgaacca gatttcaggc     2880
   cttctttcag agccatcata cgagatctta acagtttgtt tactccagat tatgaactat     2940
   taacagaaaa tgacatgtta ccaaatatga ggataggtgc cctagggttt tctggtgcct     3000
   ttgaagaccg ggatcctaca cagtttgaag agagacattt gaaatttcta cagcaacttg     3060
   gcaagggtaa ttttgggagt gtggagatgt gccggtatga ccctctacag gacaacactg 3120
   gggaggtggt cgctgtaaaa aagcttcagc atagtactga agagcaccta agagactttg 3180
   aaagggaaat tgaaatcctg aaatccctac agcatgacaa cattgtaaag tacaagggag 3240
   tgtgctacag tgctggtcgg cgtaatctaa aattaattat ggaatattta ccatatggaa 3300
   gtttacgaga ctatcttcaa aaacataaag aacggataga tcacataaaa cttctgcagt 3360
   acacatctca gatatgcaag ggtatggagt atcttggtac aaaaaggtat atccacaggg 3420
   atctggcaac gagaaatata ttggtggaga acgagaacag agttaaaatt ggagattttg 3480
   ggttaaccaa agtcttgcca caagacaaag aatactataa agtaaaagaa cctggtgaaa 3540
   gtcccatatt ctggtatgct ccagaatcac tgacagagag caagttttct gtggcctcag 3600
   atgtttggag ctttggagtg gttctgtatg aacttttcac atacattgag aagagtaaaa 3660
   gtccaccagc ggaatttatg cgtatgattg gcaatgacaa acaaggacag atgatcgtgt 3720
   tccatttgat agaacttttg aagaataatg gaagattacc aagaccagat ggatgcccag 3780
   atgagatcta tatgatcatg acagaatgct ggaacaataa tgtaaatcaa cgcccctcct 3840
   ttagggatct agctcttcga gtggatcaaa taagggataa catggctgga tgaaagaaat 3900
   gaccttcatt ctgagaccaa agtagattta cagaacaaag ttttatattt cacattgctg 3960
   tggactatta ttacatatat cattattata taaatcatga tgctagccag caaagatgtg 4020
   aaaatatctg ctcaaaactt tcaaagttta gtaagttttt cttcatgagg ccaccagtaa 4080
   aagacattaa tgagaattcc ttagcaagga ttttgtaaga agtttcttaa acattgtctg 4140
   ttaacatcac tcttgtctgg caaaagaaaa aaaatagact ttttcaactc agctttttga 4200
   gacctgaaaa aattattatg taaattttgc aatgttaaag atgcacagaa tatgtatgta 4260
   tagtttttac cacagtggat gtataatacc ttggcatctt gtgtgatgtt ttacacacat 4320
   gagggctggt gttcattaat actgttttct aatttttcca tagttaatct ataattaatt 4380
   acttcactat acaaacaaat taagatgttc agataattga ataagtacct ttgtgtcctt 4440
   gttcatttat atcgctggcc agcattataa gcaggtgtat acttttagct tgtagttcca 4500
   tgtactgtaa atatttttca cataaaggga acaaatgtct agttttattt gtataggaaa 4560
   tttccctgac cctaaataat acattttgaa atgaaacaag cttacaaaga tataatctat 4620
   tttattatgg tttcccttgt atctatttgt ggtgaatgtg ttttttaaat ggaactatct 4680
   ccaaattttt ctaagactac tatgaacagt tttcttttaa aattttgaga ttaagaatgc 4740
   caggaatatt gtcatccttt gagctgctga ctgccaataa cattcttcga tctctgggat 4800
   ttatgctcat gaactaaatt taagcttaag ccataaaata gattagattg ttttttaaaa 4860
   atggatagct cattaagaag tgcagcaggt taagaatttt ttcctaaaga ctgtatattt 4920
   gaggggtttc agaattttgc attgcagtca tagaagagat ttatttcctt tttagagggg 4980
   aaatgaggta aataagtaaa aaagtatgct tgttaatttt attcaagaat gccagtagaa 5040
   aattcataac gtgtatcttt aagaaaaatg agcatacatc ttaaatcttt tcaatta 5097
<210> 2
   <211> 1132
   <212> PRT
   <213> Homo sapiens
<400> 2
<210> 3
   <211> 128
   <212> DNA
   <213> Homo sapiens
<400> 3
   ataaatcaaa ccttctagtc ttcagaacga atggtgtttc tgatgtacca acctcaccaa 60
   cattacagag gcctactcat atgaaccaaa tggtgtttca caaaatcaga aatgaagatt 120
   tgatattt 128
<211> 135
   <212> DNA
   <213> Homo sapiens
<400> 4
<210> 5
   <211> 88
   <212> DNA
   <213> Homo sapiens
<400> 5
   tctttctttg aagcagcaag tatgatgagc aagctttctc acaagcattt ggttttaaat 60
   tatggagtat gtgtctgtgg agacgaga 88
<210> 6
   <211> 38
   <212> DNA
   <213> Artificial Sequence
<220>
   <223> Description of Artificial Sequence: Synthetic primer
<400> 6
   tgtaaaacga cggccagtct aaatgctgtc ccccaaag 38
<210> 7
   <211> 38
   <212> DNA
   <213> Artificial Sequence
<220>
   <223> Description of Artificial Sequence: Synthetic primer
<400> 7
   caggaaacag ctatgacccc atgccaactg tttagcaa 38
<210> 8
   <211> 11
   <212> DNA
   <213> Homo sapiens
<400> 8
   cgtackaaga g 11
<210> 9
   <211> 11
   <212> DNA
   <213> Homo sapiens
<400> 9
   actacrgtca a 11
<210> 10
   <211> 39
   <212> DNA
   <213> Homo sapiens
<400> 10
   aaactattca gagwywttct kkkwmrcrrs wwkkwwraw 39
<210> 11
   <211> 14
   <212> DNA
   <213> Homo sapiens
<400> 11
   atattcyggt tcag 14